(19) Europäisches Patentamt
European Patent Office
Office européen des brevets

(11) EP 0 638 651 B1

(12) **EUROPÄISCHE PATENTSCHRIFT**

(45) Veröffentlichungstag und Bekanntmachung des Hinweises auf die Patenterteilung:
**20.09.2000 Patentblatt 2000/38**

(51) Int. Cl.[7]: **C12Q 1/52**, C12Q 1/32, C12Q 1/37

(21) Anmeldenummer: **94111561.0**

(22) Anmeldetag: **25.07.1994**

(54) **Verfahren und Mittel zur enzymatischen Bestimmung von Aspartam**

Method and means for the enzymatic determination of aspartame

Méthode et moyens pour la détermination enzymatique d'aspartame

(84) Benannte Vertragsstaaten:
**BE CH DE FR GB IT LI NL**

(30) Priorität: **06.08.1993 DE 4326419**

(43) Veröffentlichungstag der Anmeldung:
**15.02.1995 Patentblatt 1995/07**

(73) Patentinhaber: **MERCK PATENT GmbH**
**64293 Darmstadt (DE)**

(72) Erfinder: **Gromes, Reiner, Dr.**
**D-64823 Gross-Umstadt (DE)**

(56) Entgegenhaltungen:
**EP-A- 0 476 549**

- **CHEMICAL ABSTRACTS, vol. 118, no. 1, 4.Januar 1993 Columbus, Ohio, US; abstract no. 5803, W. HUMMEL ET AL.: "Enzyme-catalyzed determination of the sweetener aspartame" Seite 628; Spalte 1; XP002046109 & GIT FACHZ. LAB., Bd. 36, Nr. 3, 1992, Seiten 199-204,**
- **H. U. BERGMEYER (EDITOR-IN-CHIEF): "Methods of Enzymatic Analysis, Volume VIII" 1985 , VERLAG-CHEMIE. , WEINHEIM, DE. XP002046108 014048 * Seite 350 - Seite 357 ***

EP 0 638 651 B1

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist. (Art. 99(1) Europäisches Patentübereinkommen).

## Beschreibung

Die Erfindung betrifft Verfahren und Mittel zur enzymatischen Bestimmung von Aspartam in wäßrigen Lösungen.

Aspartam ist ein Dipeptidester aus L-Asparaginsäure und L-Phenylalaninmethylester. Aspartam ist etwa 200mal süßer als Zucker und wird wegen seiner hohen Süßkraft in vielen Lebensmitteln eingesetzt.

Die Einsatzmöglichkeiten des Sußstoffes sind jedoch wegen seiner nicht sehr hohen Stabilität begrenzt. Aspartam ist nur in fester Form stabil, in wäßrigen Lösungen ist die Stabilität bei pH-Werten von 4 bis 5 am größten. Die Stabilität ist außerdem abhängig von der Lagertemperatur und der Lagerzeit. Bei nicht sachgemäßer Verarbeitung oder Lagerung können aus Aspartam verschiedene Abbauprodukte entstehen, z.B. Asparaginsäure, Phenylalanin, Phenylalaninmethylester oder Aspartylphenylalanin.

Aus diesem Grunde ist die Bestimmung von Aspartam, insbesondere bei der Qualitätskontrolle von Lebensmitteln, aber auch als Prozeßkontrolle bei der Herstellung und Verarbeitung von Lebensmitteln von großem Interesse.

Aspartam kann nach verschiedenen chromatographischen Verfahren (HPLC, DC, GC) bestimmt werden. Diese Bestimmungsverfahren erfordern in der Regel besondere Probenvorbereitungsschritte und sind daher sehr zeitaufwendig. Außerdem ist der apparative Aufwand für die Bestimmung recht erheblich.

Eine Alternative stellen enzymatische Bestimmungsverfahren dar. Sie sind einfach und schnell und ohne größeren apparativen Aufwand mit ausreichender Empfindichkeit durchführbar. Bei Verwendung spezifischer Enzyme kann die Probe ohne besonders Aufarbeitungsschritte direkt in das Testsystem eingesetzt werden. Enzymatische Verfahren zur Bestimmung von Aspartam in Verbindung mit Elektroden oder Sensoren sind z.B. aus Anal. Chim. Acta 234, 465 (1990) bekannt. Diese Verfahren haben jedoch den Nachteil, daß für die Detektion des Meßsignals Elektroden oder Sensoren benötigt werden, die nur begrenzt stabil und zum Teil schwer verfügbar sind.

Aus Analyst 115, 435 (1990) ist ein enzymatisches Verfahren zur Bestimmung von Aspartam bekannt, bei dem Aspartam nach enzymatischer Hydrolyse mit einer Peptidase mit Alkoholoxidase umgesetzt wird, wobei Formaldehyd entsteht. Formaldehyd wird mit Aminopent-3-en-2-on umgesetzt, das Produkt über eine Säule gereinigt und das Eluat photometrisch gemessen. Die Nachteile dieses Verfahrens sind der relativ hohe Zeitbedarf für eine Analyse wegen des Reinigungsschrittes für das Reaktionsprodukt sowie das zusätzlich benötigte Säulenmaterial.

In DE 4029296 wird ein Verfahren zur Bestimmung von Aspartam beschrieben, bei dem die drei an der Reaktion beteiligten Enzyme räumlich durch Immobilisierung voneinander getrennt sind. Die enzymatischen Reaktionen werden in aufeinanderfolgenden Enzymsäulen als Fließ-Injektions-Analyse durchgeführt, wobei ein Reaktionsprodukt fluorimetrisch bestimmt wird. Die Nachteile dieses Verfahrens sind in der erforderlichen Immobilisierung der drei an der Bestimmung beteiligten Enzyme, sowie der begrenzten Stabilität und Verfügbarkeit der immobilisierten Enzyme zu sehen. Für die Hydrolyse von Aspartam werden zwei spezielle Proteasen (Pronase E und Chymotrypsin) eingesetzt, um das gewünschte Reaktionsprodukt Phenylalanin freizusetzen. Photometrische Küvettentests auf der Basis dieser Enzyme werden nach diesem Stand der Technik für unmöglich gehalten, weil zwei der eingesetzten Enzyme Proteasen sind, die sowohl die Phenylalanin-Dehydrogenase als auch sich selbst gegenseitig inaktivieren würden. Eine Limitierung erfährt dieses Verfahren auch bei Proben, die fluoreszierende Verbindungen enthalten, weil bei diesen Proben eine Auswertung mittels Fluoreszenzphotometrie nur mit zusätzlichen Probenvorbereitungsschritten möglich ist (GIT Fachz. Lab. 3/93, 199 (1992)).

Der vorliegenden Erfindung liegt die Aufgabe zugrunde, ein Verfahren und Mittel zur spezifischen Bestimmung von Aspartam in Lebensmitteln und biologischen Proben zur Verfügung zu stellen, das auf der Basis eines einfachen photometrischen Tests durchführbar ist.

Gegenstand der Erfindung ist ein Verfahren zur enzymatischen Bestimmung von Aspartam in wäßrigen Lösungen, das dadurch gekennzeichnet ist, daß man die Probelösung in Gegenwart von im wesentlichen Oxoglutarat, Glutamat-Oxalacetat-Transaminase (GOT), Malat-Dehydrogenase (MDH), NADH und einer Protease inkubiert und die Abnahme der NADH-Extinktion photometrisch mißt.

Ein weiterer Gegenstand der Erfindung ist ein Mittel zur enzymatischen Bestimmung von Aspartam in wäßrigen Lösungen, enthaltend im wesentlichen Oxoglutarat, GOT, MDH, NADH und eine Protease.

Für die enzymatische Bestimmung der Aminosäure L-Asparaginsäure gibt es in der Literatur eine Reihe von Beispielen. Ein Verfahren auf der Basis eines gekoppelten UV-Testes wird in Bergmeyer "Methods of Enzymatic Analysis", Vol. VIII, 350-357 (1985) beschrieben:

(1) $$\text{Asparaginsäure} + \text{Oxoglutarat} \xrightleftharpoons{\text{GOT}} \text{Oxalacetat} + \text{Glutaminsäure}$$

(2) $$\text{Oxalacetat} + \text{NADH} \xrightarrow{\text{MDH}} \text{Malat} + \text{NAD}^+$$

Asparaginsäure kann aus Aspartam durch verschiedene Proteasen freigesetzt werden:

(3) $$\text{Aspartam} \xrightleftharpoons{\text{Protease}} \text{Asparaginsäure} + \text{Phenylalaninmethylester}$$

Überraschenderweise wurde gefunden, daß bei geeigneter Wahl der Reaktionsbedingungen die Bestimmung von Aspartam durch Hydrolyse mit einer Protease und Nachweis der entstandenen Asparaginsäure nach den Gleichungen (1) und (2) in einem einzigen Reaktionsansatz möglich ist. Proben und Aspartamstandards werden dabei unter gleichen Reaktionsbedingungen (Reagenzien, Volumina, Zeit, Temperatur) nebeneinander inkubiert und die Extinktionen vor Zugabe der Protease ($E_1$) und eine bestimmte Zeit, z.B. 20 Minuten nach Zugabe der Protease ($E_2$) bei 340 oder 365 nm bestimmt. Die Extinktionsdifferenz ($E_1$-$E_2$) ist der Aspartamkonzentration im Bereich von 0-500 mg/l Aspartam direkt proportional. Die Bestimmung des Aspartamgehaltes unbekannter Proben erfolgt somit durch Vergleich der Extinktionsdifferenzen von Proben und einer Standardlösung mit bekannter Aspartamkonzentration oder verschieden konzentrierter Aspartamstandards über eine Kalibrationskurve.

Durch die Reihenfolge der Reaktionen (1, 2, 3) ist die Spezifität für Aspartam gewährleistet. Große Probenzahlen lassen sich parallel nebeneinander bestimmen (ca. 50 Proben). Die Sensitivität ist vergleichbar mit der bereits beschriebener Verfahren. Der Vorteil des erfindungsgemäßen Verfahrens liegt in der Einfachheit der Durchführung ohne größeren apparativen Aufwand sowie in der Stabilität der verwendeten Reagenzien. Eine zusätzliche Erleichterung erfährt das Verfahren durch die Bereitstellung von gebrauchsfertigen Reagenzien in fester Form (z.B. Tabletten) zu einem Testkit. Prinzipiell ist das erfindungsgemäße Verfahren auch als Mikrotiterplattentest oder auf einem Autoanalyser durchführbar. Zur Empfindlichkeitssteigerung kann auch ein Fluoreszenzdetektor eingesetzt werden.

Neben Oxoglutarat, NADH und den Enzymen enthält das erfindungsgemäße Mittel vorzugsweise noch Pyridoxal-5-phosphat, das als Coenzym die optimale Aktivität der Transaminase (GOT) bewirkt.

Das erfindungsgemäße Verfahren wird vorzugsweise in Gegenwart eines Puffers durchgeführt, der es erlaubt, die Reaktionen in einem optimalen pH-Bereich von etwa 6,5-8,5, vorzugsweise bei einem pH-Wert von 7,5 ablaufen zu lassen. Geeignete Puffer sind solche, die den Reaktionsablauf nicht stören wie z.B. Tris/HCl-, HEPES-, Imidazol-, Triethanolamin, PIPES-Puffer, vorzugsweise Tris/HCl-Puffer. Die Pufferkonzentration sollte im Bereich von etwa 50 bis 500 mM liegen.

Die Konzentrationen der übrigen Bestandteile sollten in folgenden Bereichen liegen: Oxoglutarat etwa 5 bis 20 mM, Pyridoxal-5-phosphat etwa 0 bis 1 mM, NADH etwa 150 bis 240 µM, GOT etwa 1 bis 10 KU/l, MDH etwa 10 bis 100 KU/l.

Zur Hydrolyse von Aspartam sind die im Handel erhältlichen Proteasen wie Pronase E, Aminopeptidase M, Aminopeptidase K, Leucinaminopeptidase, Alcalase, vorzugsweise Pronase E, geeignet. Im Falle von Pronase E sollte die Konzentration im Testansatz etwa 0,2 bis 2 mg betragen, vorzugsweise 1 mg/Ansatz,. Mit weniger aktiven Proteasen erhält man durch Erhöhung der Konzentration und/oder Verlängerung der Inkubationszeit analoge Ergebnisse.

Die Bestimmung von Aspartam erfolgt erfindungsgemäß in Gegenwart aller Reaktionspartner in einem einzigen Reaktionsgefäß. Vorzugsweise wird die Inkubation in zwei Schritten durchgeführt, wobei die Inkubation mit der Protease im zweiten Schritt erfolgt. Die Probelösung wird dazu mit einer Reaktionslösung versetzt, die alle erforderlichen Reagenzien außer der Protease enthält. Nach einer Wartezeit von wenigen Minuten wird die Extinktion $E_1$ bestimmt. Danach erfolgt die Zugabe der Proteaselösung, und nach einer Inkubationszeit von 2 bis 60 Minuten, vorzugsweise etwa 20 Minuten, bei 15 bis 40 °C wird die Extinktion $E_2$ bestimmt. Meßgröße ist die bei 340 oder 365 nm gemessene Abnahme der NADH-Konzentration. Die Auswertung erfolgt anhand einer Kalibrationskurve.

Das efindungsgemäße Mittel kommt vorzugsweise in Form eines Testkits zur Anwendung, der die gebrauchsfertigen Reagenzien z.B. in Form von Tabletten oder Lyophilisaten enthält. Oxoglutarat, Pyridoxal-5-phosphat, NADH und die Protease liegen z.B. als Tabletten vor, Aspartam als Lyophilisat und die Enzyme GOT und MDH in Form einer Suspension. Enthält die zu untersuchende Probe Proteine, so ist es vorteilhaft, diese mit den üblichen Proteinfällungsreagenzien (z.B. Carrez-Reagenz: Kaliumhexacyanoferrat, Zinksulfat) auszufällen und durch Zentrifugation

aus der Probelösung zu entfernen.

Beispiel 1

Zu 0,2 ml Probelösung (100; 200; 300; 400; 500 mg Aspartam/l; Wasser im Leerwert) werden 2,0 ml einer Reaktionslösung mit folgenden Reagenzien (Konzentration im Reaktionsansatz: 100 mM Tris/HCl, pH 7,5; 11,3 mM Oxoglutarat; 160 µM NADH; 91 µM Pyridoxal-5-phosphat; 6,2 KU/l GOT; 49,6 KU/l MDH) zugegeben, 5 Minuten gewartet und die Extinktion ($E_1$) bestimmt. Danach erfolgt die Zugabe von 0,2 ml Pronase-E-Lösung (5 mg Pronase E in 1 ml 125 mM Tris/HCl, pH 7,5). Nach 20 Minuten wird die Extinktion ($E_2$) bestimmt. Die Auswertung erfolgt durch Aufragen der Extinktionsdifferenzen (ΔE) gegen die Standardkonzentrationen:

$$\Delta E_{LW} = E_2 - E_1 \qquad \text{Lw = Leerwerte}$$

$$\Delta E_{Pr} = E_2 - E_1 \qquad \text{Pr = Probe / Standard}$$

$$\Delta E = \Delta E_{Pr} - \Delta E_{Lw}$$

Unter den beschriebenen Reaktionsbedingungen besteht zwischen dem Meßsignal (ΔE) und der Aspartamkonzentration in der Probe ein linearer Zusammenhang im Bereich von 0-500 mg/l Aspartam. Eine Kalibrationskurve für diesen Bereich ist in Fig 1 dargestellt.

Beispiel 2

a) Reagenzien für 50 Testansätze

Reagenz 1: 30 ml Tris/HCl-Pufferlösung, pH 7,5 (500 mM)
Reagenz 2: 5 Oxoglutarat/Pyridoxal-5-phosphat-Tabletten (63,4 mg/0,6 mg/Tablette)
Reagenz 3: 10 NADH-Tabletten (1,42 mg/Tablette)
Reagenz 4: 1 ml GOT/MDH-Suspension (750 U GOT/6000 U MDH/ml)
Reagenz 5: 5 Pronase-E-Tabletten (10 mg/Tablette)
Reagenz 6: 5 mg Aspartam-Lyophilisat

Alle Reagenzien sind bei 4 °C mindestens ein Jahr stabil.

b) Standardlösungen

Zur Herstellung von Standardlösungen für eine Kalibrationskurve wird Reagenz 6 in 10 ml bidest. Wasser gelöst und wie folgt verdünnt:

| Aspartam-Endkonz. (mg/l) | Reagenz 6 (ml) | Wasser (ml) |
|---|---|---|
| 50 | 0,25 | 2,25 |
| 100 | 0,50 | 2,00 |
| 200 | 1,00 | 1,50 |
| 300 | 1,50 | 1,00 |
| 400 | 2,00 | 0,50 |
| 500 | 2,50 | 0,00 |

Die verdünnten Standardlösungen sollten portionsweise bei -20 °C gelagert werden.

c) Reaktionslösungen

Zur Herstellung von **Reaktionslösung A** werden in einem Becherglas eine Tablette Reagenz 2 und zwei

Tabletten Reagenz 3 in 5 ml Reagenz 1 und 15,5 ml bidest. Wasser gelöst, anschließend werden 0,2 ml Reagenz 4 zugesetzt und vorsichtig gerührt.

Zur Herstellung von **Reaktionslösung B** wird eine Tablette Reagenz 5 in 0,5 ml Reagenz 1 und 1,6 ml bidest. Wasser gelöst.

Die beiden Reaktionslösungen sind jeweils ausreichend für 10 Reaktionsansätze. Sie sollten immer frisch hergestellt und direkt nach der Herstellung verbraucht werden.

d) Pipettierschema

Die Zugabe der beiden Reaktionslösungen in die Küvetten mit Probe-bzw. Standardlösungen sollte im Zeittakt erfolgen

| | Reagenzienleerwert | Standard/Probe |
|---|---|---|
| Wasser | 0,2 ml | - |
| Standard/Probe | - | 0,2 ml |
| Lösung A | 2 ml | 2 ml |
| 5 Minuten warten, $E_1$ messen | | |
| Lösung B | 0,2 ml | 0,2 ml |
| 20 Minuten warten, $E_2$ messen | | |
| Wellenlänge: 340 nm oder 365 nm (Raumtemperatur)<br>Schichtdicke (Küvette): 1 cm | | |

e) Auswertung

Die Auswertung kann über die Steigung der Kalibrationskurve oder durch Vergleich der Extinktion einer unbekannten Probe ($E_P$) mit der Extinktion eines Standards ($E_S$) bekannter Konzentration unter Berücksichtigung des Leerwertes ($E_L$) nach folgenden Gleichungen erfolgen:

$$\Delta E_{S/P} = (E_{1S/P} - E_{2S/P}) - (E_{1L} - E_{2L})$$

$$C_P = \frac{\Delta E_P}{\Delta E_S} \cdot C_S$$

Beispiel 3

Bestimmung von Aspartam in Lebensmitteln Vergleich der HPLC-Methode mit dem erfindungsgemäßen Verfahren

Zur Bestimmung des Aspartamgehalts in verschiedenen Lebensmitteln wurden die Proben sowohl für die HPLC-Bestimmung als auch für den Enzymtest wie folgt vorbereitet:

- Die Getränkeproben wurden durch kurzes Schütteln entgast und anschließend filtriert;
- Bonbons, Streusüße und Süßstofftablette wurden gewogen, eine definierte Menge in 10 ml bidest. Wasser aufgelöst und anschließend filtriert.

Aus den so hergestellten Probelösungen wurde der Aspartamgehalt nach der HPLC-Methode (Amtl. Sammlung von Untersuchungsverfahren nach § 35 LMBG, L 32, 13-1 vom Dez. 1989) und nach dem Verfahren gemäß der Erfindung bestimmt. Die Ergebnisse aus den beiden Bestimmungsmethoden sind in der nachstehenden Tabelle gegenübergestellt. Der Vergleich der Meßwerte aus den beiden Methoden zeigt, daß die beschriebene neue enzymatische Methode zur Bestimmung des Aspartamgehaltes in Lebensmitteln sehr gut geeignet ist. Für die Messung der 10 Proben nach der HPLC-Methode wurde etwa die fünffache Zeit im Vergleich zur enzymatischen Bestimmung benötigt.

| Probe | Aspartam [mg/l] | |
|---|---|---|
| | HPLC-Methode | Enzymatische Methode |
| Getränk 1 | 31 | 34 |
| Getränk 2 | 426 | 429 |
| Getränk 3 | 471 | 487 |
| Getränk 4 | 19 | 23 |
| Getränk 5 | 18 | 21 |
| Süßstofftablette 1 | 163 | 161 |
| Süßstofftablette 2 | 178 | 179 |
| Streusüße | 267 | 268 |
| Bonbon | 96 | 95 |
| Joghurt | 43 | 39 |

## Patentansprüche

1. Verfahren zur enzymatischen Bestimmung von Aspartam in wäßrigen Lösungen, dadurch gekennzeichnet, daß man die Probelösung in Gegenwart von Oxoglutarat, Glutamat-Oxalacetat-Transaminase (GOT), Malat-Dehydrogenase (MDH), NADH und einer Protease inkubiert und die Abnahme der NADH-Extinktion photometrisch mißt.

2. Verfahren nach Anspruch 1, dadurch gekennzeichnet, daß es in Gegenwart von Pyridoxal-5-phosphat durchgeführt wird.

3. Verfahren nach den Ansprüchen 1 und 2, dadurch gekennzeichnet, daß die Bestimmung in Gegenwart aller Reaktionspartner in einem einzigen Reaktionsgefäß durchgeführt wird.

4. Verfahren nach den Ansprüchen 1 bis 3, dadurch gekennzeichnet, daß die Inkubation in zwei Schritten erfolgt.

5. Verfahren nach den Ansprüchen 1 bis 4, dadurch gekennzeichnet, daß die Inkubation mit der Protease im zweiten Schritt erfolgt.

6. Verfahren nach den Ansprüchen 1 bis 5, dadurch gekennzeichnet, daß als Protease Pronase E verwendet wird.

7. Testkit zur enzymatischen Bestimmung von Aspartarn in wäßrigen Lösungen, enthaltend Oxoglutarat, GOT, MDH, NADH und eine Protease.

8. Testkit nach Anspruch 7, dadurch gekennzeichnet, daß Oxoglutarat, ggf. Pyridoxal-5-phosphat, NADH und die Protease in fester Form vorliegen.

## Claims

1. Method for the enzymic determination of Aspartame in aqueous solutions, characterized in that the sample solution is incubated in the presence of oxoglutarate, glutamate-oxaloacetate transaminase (GOT), malate dehydrogenase (MDH), NADH and a protease, and the decrease in the NADH extinction is measured photometrically.

2. Method according to Claim 1, characterized in that it is carried out in the presence of pyridoxal 5-phosphate.

3. Method according to Claims 1 and 2, characterized in that the determination is carried out in the presence of all the

reagents in a single reaction vessel.

4. Method according to Claims 1 to 3, characterized in that the incubation is carried out in two steps.

5. Method according to Claims 1 to 4, characterized in that the incubation with the protease is carried out in the second step.

6. Method according to Claims 1 to 5, characterized in that pronase E is used as the protease.

7. Test kit for the enzymic determination of Aspartame in aqueous solutions, which test kit contains oxoglutarate, GOT, MDH, NADH and a protease.

8. Test kit according to Claim 7, characterized in that oxoglutarate, where appropriate pyridoxal 5-phosphate, NADH and the protease are present in solid form.

**Revendications**

1. Procédé pour la détermination enzymatique de l'aspartame dans des solutions aqueuses, caractérisé en ce que l'on met la solution échantillon à incuber en présence d'oxoglutarate, de glutamate-oxaloacétate transaminase (GOT), de malate déshydrogénase (MDH), de NADH et d'une protéase, et on mesure photométriquement la diminution de l'extinction du NADH.

2. Procédé selon la revendication 1, caractérisé en ce qu'il est effectué en présence de pyridoxal-5-phosphate.

3. Procédé selon les revendications 1 et 2, caractérisé en ce que l'on effectue la détermination en présence de tous les partenaires réactionnels dans un seul récipient de réaction.

4. Procédé selon les revendications 1 à 3, caractérisé en ce que l'incubation s'effectue en deux étapes.

5. Procédé selon les revendications 1 à 4, caractérisé en ce que l'incubation avec la protéase s'effectue en deux étapes.

6. Procédé selon les revendications 1 à 5, caractérisé en ce que l'on utilise comme protéase la protéase E.

7. Nécessaire d'essai pour la détermination enzymatique de l'aspartame dans des solutions aqueuses contenant essentiellement de l'oxoglutarate, GOT, MDH, NADH et une protéase.

8. Nécessaire d'essai selon la revendication 7, caractérisé en ce que l'oxoglutarate, éventuellement le pyridoxal-5-phosphate, le NADH et la protéase se trouvent sous forme solide.

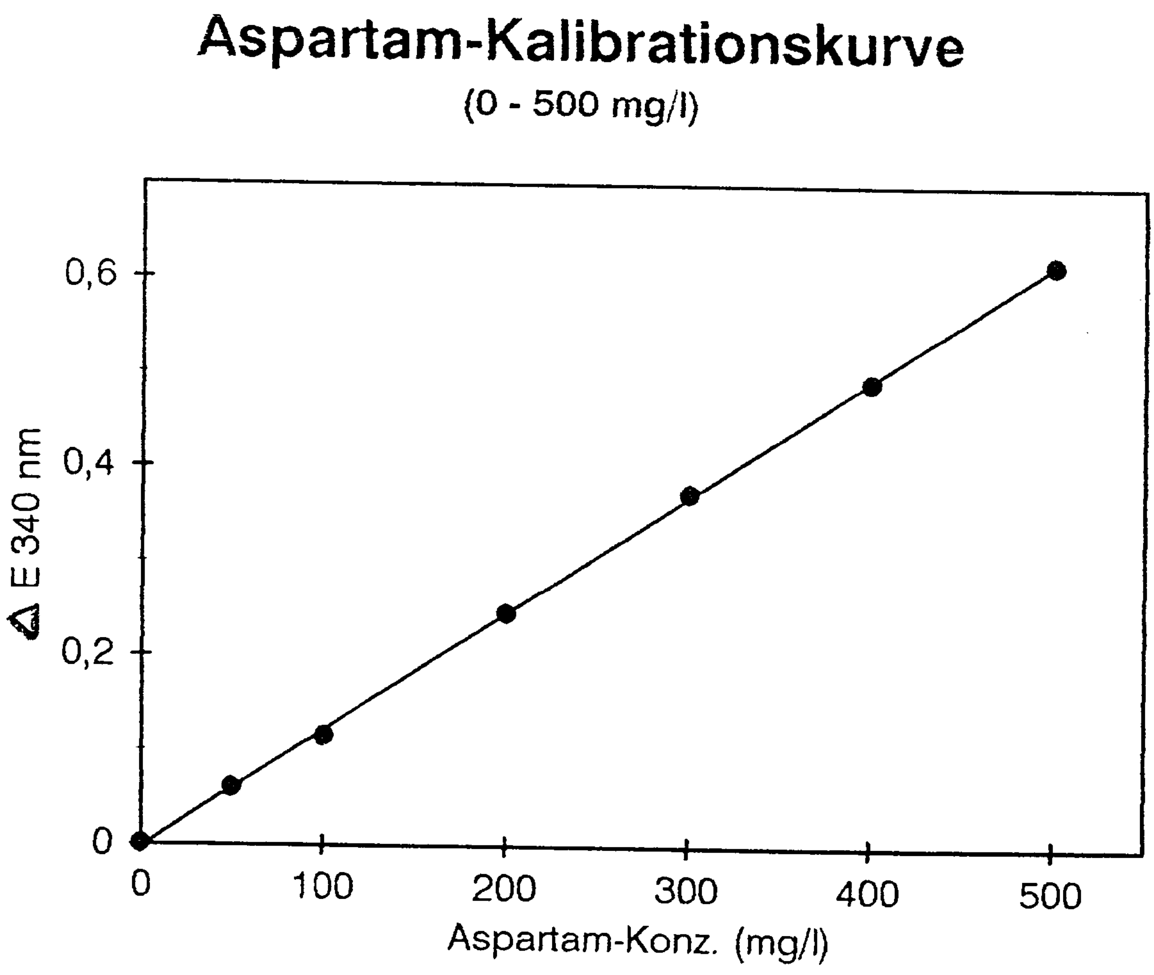
Aspartam-Kalibrationskurve
(0 - 500 mg/l)
0,6
0,4
0,2
0
Δ E 340 nm
0
100
200
300
400
500
Aspartam-Konz. (mg/l)